# EUROPEAN PATENT SPECIFICATION

(11) **EP 3 991 559 B1**
(45) Date of publication and mention of the grant of the patent: **29.07.2026**
(21) Application number: 20830791.8
(22) Date of filing: 26.06.2020
(51) Int. Cl.: A01N 63/30, A01N 25/00, A01N 25/04, A01N 25/12, A01N 25/14, A01N 25/30, A01P 3/00

(54) **USE OF A PLANT DISEASE CONTROL AGENT AND PLANT DISEASE CONTROL METHOD**
VERWENDUNG EINES MITTELS ZUR KONTROLLE VON PFLANZENKRANKHEITEN UND VERFAHREN ZUR KONTROLLE VON PFLANZENKRANKHEITEN
UTILISATION D'UN AGENT DE LUTTE CONTRE LES MALADIES DES PLANTES ET PROCÉDÉ DE LUTTE CONTRE LES MALADIES DES PLANTES

(30) Priority: 27.06.2019 JP 2019120198; 14.01.2020 JP 2020003941
(43) Date of publication of application: 04.05.2022
(73) Proprietor: SDS BIOTECH K.K., Tokyo 101-0022 (JP)
(72) Inventor: TSUKAGOSHI, Yuki, Tsukuba-shi, Ibaraki 300-2646 (JP)
(74) Representative: Vossius & Partner Patentanwälte Rechtsanwälte mbB
(86) International application number: PCT/JP2020/025223
(87) International publication number: WO 2020/262612

(56) References cited:
- WO-A1-02/35934
- WO-A1-2007/011025
- CN-A- 1 924 005
- CN-A- 102 286 383
- CN-A- 106 119 134
- CN-A- 109 593 658
- CN-B- 102 286 383
- CN-B- 104 286 032
- JP-A- 2015 093 850
- US-A- 4 818 530
- MANOCH L ET AL: "A Potential Use of Talaromyces species as Biological Agents Against Plant Pathogenic Fungi", THAI J. AGRIC. SCI., 1 January 2011 (2011-01-01), pages 81 - 91, XP093282274, Retrieved from the Internet <URL:https://www.thaiscience.info/journals/Article/TJAS/10899373.pdf>
- DETHOUP T ET AL: "Morphology and Distribution of Talaromyces flavus from Soil and Potential Use as a Biological Control Agent against Plant Pathogenic Fungi", THAI J. AGRIC. SCI., 1 January 2007 (2007-01-01), pages 37 - 50, XP093282278, Retrieved from the Internet <URL:https://www.thaiscience.info/journals/Article/TJAS/10469511.pdf>
- LEI LI-RONG ET AL: "Research advances in the structures and biological activities of secondary metabolites from Talaromyces", FRONTIERS IN MICROBIOLOGY, vol. 13, 19 August 2022 (2022-08-19), Lausanne, pages 1 - 23, XP093282275, ISSN: 1664-302X, Retrieved from the Internet <URL:https://www.frontiersin.org/journals/microbiology/articles/10.3389/fmicb.2022.984801/pdf> DOI: 10.3389/fmicb.2022.984801
- KAKVAN NIKOO; HEYDARI ASGHAR; ZAMANIZADEH HAMID REZA; REZAEE SAEED; NARAGHI LALEH: "Development of new bioformulations usingTrichodermaandTalaromycesfungal antagonists for biological control of sugar beet damping-off disease", CROP PROTECTION, vol. 53, 2013, pages 80 - 84, XP028728573
- KOCH E; ET AL: "Evaluation of commercial products for microbila control of soil-borne plant diseases", CROP PROTECTION, vol. 18, 1999, pages 119 - 125, XP002983743

## Description

### TECHNICAL FIELD

The present invention is defined by the claims. The disclosure herein generally relates to a control agent for controlling a disease of an edible plant such as a cereal or a vegetable for humans and/or animals, which disease is caused by infection, and to a disease control method using the control agent.

### BACKGROUND ART

The sharp increase in the world population has led to an increased demand for food, and hence to requirement of more efficient and productive crop cultivation in the limited cultivation area. However, the crop yield is decreasing due to the climate changes caused by global warming and the like.

At present, crop diseases are controlled by the use of chemical pesticides. However, their excessive use has led to the appearance of pathogenic microbes having drug resistance, and environmental pollution by the pesticides themselves has also become problematic. Thus, crop disease controlling methods independent of chemical pesticides, whose loads on the environment are therefore low, are important means for allowing continuous development of agriculture.

In recent years, microbial pesticides using microorganisms having control effects against plant diseases have been developed. Known examples of such microorganisms include filamentous fungi belonging to the genus *Talaromyces.* For example, Patent Document 1 (JP 3601928 B) discloses the *Talaromyces flavus* Y-9401 strain, which has a control effect against strawberry anthracnose.

Further, Patent Documents 2 to 4 describe that *Talaromyces flavus* has a control effect against tomato gray mold and powdery mildew, and Patent Documents 5 to 7 describe that it has a control effect against rice diseases.

Further, Non-patent Document 1 describes that, when a solid culture of the *Talaromyces flavus* fungus was sprayed onto a contaminated soil prepared by spraying of sclerotia of *Sclerotinia sclerotirum,* the occurrence of sclerotium rot could be controlled in soybean, rapeseed, wheat, and barley.

Non-patent Document 2 describes that occurrence of potato verticillium wilt (causative fungus, *Verticillium albo-atrum*) could be suppressed by seed disinfection by soaking in a suspension of *Talaromyces flavus* spores. This document also describes that potato verticillium wilt could be controlled also by direct seeding of non-disinfected potato in a soil treated with *Talaromyces flavus* spores.

Non-patent Document 3 describes that stem rot (causative fungus, *Sclerotinia sclerotirum*) could be controlled by spraying of a fungal suspension of *Talaromyces flavus* to potato seedlings.

Non-patent Document 4 describes that verticillium wilt (causative fungus, *Verticillium dahliae*) could be controlled by treatment of seeds or stem tubers of eggplant or potato with a simple preparation containing spores of the *Talaromyces flavus* fungus.

Non-patent Document 5 describes that damping-off due to *Rhizoctonia solani* could be controlled by treatment of sugar beet seeds with the *Talaromyces flavus* fungus.

### PRIOR ART DOCUMENTS

### [Patent Documents]

[Patent Document 1] JP 3601928 B
[Patent Document 2] JP 4226323 B
[Patent Document 3] JP 2006-124337 A
[Patent Document 4] JP 2009-221132 A
[Patent Document 5] JP 4810151 B
[Patent Document 6] JP 2014-237609 A
[Patent Document 7] JP 2015-093850 A

### [Non-patent Documents]

[Non-patent Document 1] MCLAREN D L, HUANG H C, RIMMERS R. (1996), Control of Apothecial Production of Sclerotinia sclerotiorum by Coniothyrium minitans and Talaromyces flavus. Plant Dis Vol. 80 No. 12 Page. 1373-1378
[Non-patent Document 2] Naraghi, L., Heydari, A., Rezaee, S., Razavi, M., & Jahanifar, H. (2010). Study on antagonistic effects of Talaromyces flavus on Verticillium albo-atrum, the causal agent of potato wilt disease. Crop Protection, 29(7), 658-662.
[Non-patent Document 3] OJAGHIAN Mohammad Reza (2011) Potential of Trichoderma spp. and Talaromyces flavus for biological control of potato stem rot caused by Sclerotinia sclerotiorum. Phytoparasitica Vol. 39 No. 2 Page. 185-193 [Non-patent Document 4] Nagtzaam, M. P. M., & Bollen, G. J. (1997). Colonization of roots of eggplant and potato by Talaromyces flavus from coated seed. Soil Biology and Biochemistry, 29(9-10), 1499-1507.
[Non-patent Document 5] Kakvan, N., Heydari, A., Zamanizadeh, H. R., Rezaee, S., & Naraghi, L. (2013). Development of new bioformulations using Trichoderma and Talaromyces fungal antagonists for biological control of sugar beet damping-off disease. Crop Protection, 53, 80-84.
WO 2007/011025 provides a microbial pesticide which exhibits a controlling effect on a number of diseases occurring in the stage of raising rice seedlings, has a high safety and little affects the environment.
CN109593658A describes the endophytic fungus ZXL-SZY-R-9 with antibiotic effect and its application.
CN102286383B relates to a yellow basket fungus and its application in preventing and controlling plant pathogens.
Manoch et al. (2011) Thai J. Agric. Sci., 44(2):81-91 describes the potential use of *Talaromyces* species as biological agents against plant pathogenic fungi.
Dethoup et al. (2007), Thai J. Agric. Sci., 40(1-2):37-50 relates to the morphology and distribution of *Talaromyces flavus* from soil and its potential use as a biological control agent against plant pathogenic fungi.

### SUMMARY OF THE INVENTION

### PROBLEMS TO BE SOLVED BY THE INVENTION

As described above, filamentous fungi belonging to the genus *Talaromyces* have been known to be useful as disease control agents. However, their application to a wider range of types of plants and diseases has been demanded.

Herein disclosed but not claimed is a microbial preparation for a plant selected from the group consisting of Brassicaceae plant; beans; maize; wheat variety; tubers and roots; and sugar beet; which microbial preparation is used for controlling a disease caused by a fungus selected from the group consisting of fungi belonging to the genus *Rhizoctonia,* fungi belonging to the genus *Fusarium,* fungi belonging to the genus *Pythium,* fungi belonging to the genus *Microdochium,* fungi belonging to the genus *Tilletia,* fungi belonging to the genus *Ustilago,* fungi belonging to the genus *Streptomyces,* fungi belonging to the genus *Erwinia,* and fungi belonging to the genus *Aphanomyces.*

### MEANS FOR SOLVING THE PROBLEMS

In order to solve the above problem, the present inventors intensively studied and found that, by treating seeds, seedlings, soil, and/or the like of a plant selected from the group consisting of Brassicaceae plant; beans; maize; wheat variety; tubers and roots; and sugar beet; with a filamentous fungus belonging to the genus *Talaromyces,* stable and efficient control of diseases caused by fungi belonging to the genus *Rhizoctonia,* fungi belonging to the genus *Fusarium,* fungi belonging to the genus *Pythium,* fungi belonging to the genus *Microdochium,* fungi belonging to the genus *Tilletia,* fungi belonging to the genus *Ustilago,* fungi belonging to the genus *Streptomyces,* fungi belonging to the genus *Erwinia,* and fungi belonging to the genus *Aphanomyces* can be achieved. The present invention is defined by the appended claims.

The present invention can be summarized as follows.
[1] Use of an agent comprising a filamentous fungus belonging to the genus *Talaromyces* for controlling a disease for a plant selected from the group consisting of cabbage; soybeans; maize; wheat variety; and potato, wherein
   when said plant is cabbage said disease is caused by fungi belonging to the genus *Rhizoctonia,*
   when said plant is maize said disease is caused by fungi belonging to the genus *Pythium,*
   when said plant is wheat variety said disease is caused by fungi belonging to the genus *Pythium,*
   when said plant is soybean said disease is caused by fungi belonging to the genus *Fusarium,* and
   when said plant is potato said disease is caused by bacteria belonging to the genus *Streptomyces* or fungi belonging to the genus *Rhizoctonia,* and
   wherein the *Talaromyces flavus* is *Talaromyces flavus* Y-9401 strain (FERM BP-10642).
[2] The use according to 1, wherein said agent comprises the *Talaromyces flavus* Y-9401 strain (FERM BP-10642) in an amount of 1×10⁶ to 1×10¹² colony-forming units/g.
[3] The use according to any one of 1 to 2, wherein said agent further comprising a surfactant and/or an excipient.
[4] The use according to 3, wherein the surfactant is one or more surfactants selected from the group consisting of fatty acid soaps, alkyl ether carboxylic acid, N-acylamino acid, alkyl benzene sulfonate, alkyl naphthalene sulfonate, alpha-olefin sulfonate, dialkyl sulfosuccinate salt, higher alcohol sulfate salt, alkyl ether sulfate, polyoxyethylene alkylphenyl ether sulfate, fatty acid alkylol amide sulfate salt, alkyl ether phosphate salt, alkyl phosphate salt, aliphatic amine salt, aliphatic quaternary ammonium salt, benzalkonium salt, benzethonium chloride, pyridinium salt, imidazolinium salt, polyoxyethylene alkyl ether, polyoxyethylene alkylphenyl ether, polyoxyethylene polyoxypropylene block polymer, polyoxyethylene polyoxypropylene alkyl ether, polyoxyethylene glycerin fatty acid ester, polyoxyethylene castor oil, polyoxyethylene sorbitan fatty acid ester, polyoxyethylene sorbitol fatty acid ester, polyethylene glycol fatty acid ester, fatty acid monoglyceride, polyglycerin fatty acid ester, sorbitan fatty acid ester, fatty acid alkanolamide, polyoxyethylene fatty acid amide, polyoxyethylene alkylamine, alkylamine oxide, carboxybetaine, aminocarboxylate, and imidazolinium betaine.
[5] The use according to 3 or 4, wherein the excipient is one or more excipients selected from the group consisting of:
   fine mineral powders selected from the group consisting of kaolin clay, pyrophyllite clay, bentonite, montmorillonite, diatomaceous earth, synthetic hydrated silicon oxide, acid clay, talc, clay, ceramic, quartz, sericite, vermiculite, perlite, Oya stone, anthracite, limestone, stone coal, and zeolite;
   inorganic compounds selected from the group consisting of common salt, carbonates, sulfates, nitrates, and urea;
   organic matters selected from the group consisting of chaff, bran, crab shells, shrimp shells, krill powder, rice lees, wheat flour, corn cobs, peanut shells, bone meal, fish meal, lees powder, wood flour, charcoal, kuntan charcoal, bark charcoal, rice husk charcoal, herbal/wooden charcoal, peat moss, attapulgite, dried dung, activated carbon, oil cake, and starch and hydrolysates thereof;
   saccharides selected from the group consisting of D-sorbitol, lactose, maltitose, glucosamine, and oligosaccharides;
   oils selected from the group consisting of vegetable oils, animal oils, and mineral oils; and
   synthetic water-soluble polymers.
[6] The use according to any one of 3 to 5, wherein said agent comprises the *Talaromyces flavus* Y-9401 strain (FERM BP-10642) at 1 to 90% by mass, the surfactant at 1 to 15% by mass, and the excipient at 0 to 98% by mass.
[7] The use according to any one of 3 to 5, wherein said agent comprises *Talaromyces flavus* Y-9401 strain (FERM BP-10642) at 2 to 46% by mass, the surfactant at 2 to 15% by mass, and the excipient at 39 to 96% by mass.
[8] The use according to any one of 1 to 7, wherein said agent is formulated as any of a powder, a granule, an emulsion, a wettable powder, a flowable formulation, and a coating agent.
[9] A method of controlling a disease of a plant, the method comprising:
   treating seeds, seedlings, plant bodies, seedling culture soil, seedling culture medium, cultivation soil, and/or cultivation medium of the plant using an agent comprising *Talaromyces flavus* Y-9401 strain (FERM BP-10642);
   wherein the plant is selected from the group consisting of cabbage; soybeans; maize; wheat variety; and potato; and
   when said plant is cabbage said disease is caused by fungi belonging to the genus *Rhizoctonia,*
   when said plant is maize said disease is caused by fungi belonging to the genus *Pythium,*
   when said plant is wheat variety said disease is caused by fungi belonging to the genus *Pythium,*
   when said plant is soybean said disease is caused by fungi belonging to the genus *Fusarium,* and
   when said plant is potato said disease is caused by bacteria belonging to the genus *Streptomyces* or fungi belonging to the genus *Rhizoctonia.*

### EFFECT OF THE DISCLOSURE

According to the present disclosure, by treating seeds, seedlings, soil, and/or the like of a plant selected from the group consisting of Brassicaceae plant; beans; maize; wheat variety; tubers and roots; and sugar beet; with a filamentous fungus belonging to the genus *Talaromyces,* a high control effect can be produced against diseases such as damping-off caused by fungi belonging to the genus *Rhizoctonia,* fungi belonging to the genus *Fusarium,* fungi belonging to the genus *Pythium,* fungi belonging to the genus *Microdochium,* fungi belonging to the genus *Tilletia,* fungi belonging to the genus *Ustilago,* fungi belonging to the genus *Streptomyces,* fungi belonging to the genus *Erwinia,* fungi belonging to the genus *Aphanomyces.* According to the claimed invention, *Rhizoctonia* is controlled in cabbage and potato, *Pythium* is controlled in maize and wheat, *Fusarium* is controlled in soybean and *Streptomyces* are controlled in potato. According to the invention, the fungus of the genus *Talaromyces* used to achieve said control is *Talaromyces flavus* Y-9401.

In cases where the strain of the filamentous fungus belonging to the genus *Talaromyces* used is a fungal strain isolated from a natural source, for example, where the strain is the *Talaromyces flavus* Y-9401 strain, disease control is possible without adversely affecting the environment or the human body. *Talaromyces flavus* strains such as the Y-9401 strain are especially useful since their spores exhibit high storage stability, and since a plurality of formulations including wettable powders, granules, and flowable formulations can be selected therefor. According to the invention *Talaromyces flavus* Y-9401 is used. Where in the following other strains or species of the genus *Talaromyces* are described, these are for reference only.

### IMPLEMENTATIONS FOR CARRYING OUT THE DISCLOSURE

### <Filamentous Fungi Belonging to Genus Talaromyces>

Examples of the filamentous fungus belonging to the genus *Talaromyces* used for the disease control agent of the present disclosure include, but are not limited to, *Talaromyces flavus, Talaromyces bacillisporus, Talaromyces helicum, Talaromyces luteus,* and *Talaromyces rutundus. Talaromyces flavus* is preferred.

Preferred examples of the *Talaromyces flavus* include the *Talaromyces flavus* Y-9401 strain. The *Talaromyces flavus* Y-9401 strain was deposited with Patent Microorganisms Depositary, National Institute of Bioscience and Human-Technology, Agency of Industrial Science and Technology, Ministry of International Trade and Industry (currently International Patent Organism Depositary (IPOD), Biological Resource Center, National Institute of Technology and Evaluation) as of September 2, 1996 under the accession number FERM P-15816, and the deposition was converted to international deposition under the Budapest Treaty as of July 18, 2006 under the accession No. FERM BP-10642. According to the invention *Talaromyces flavus* Y-9401 is used.

### <Disease Control Agent>

The disease control agent of the present disclosure comprises, as an effective ingredient, the above filamentous fungus belonging to the genus *Talaromyces.* According to the invention *Talaromyces flavus* Y-9401 is used.

The form of the filamentous fungus belonging to the genus *Talaromyces* is not limited as long as the disease control effect can be produced. The fungus may be in the form of any of conidia, hyphae, asci, spores, and the like. From the viewpoint of storage stability of the disease control agent, the fungus is preferably in the form of spores.

The filamentous fungus belonging to the genus *Talaromyces* may be cultured by the same method as in the cases of ordinary filamentous fungi.

For example, the filamentous fungus belonging to the genus *Talaromyces* may be grown by a liquid culture method such as reciprocation liquid culture or jar fermenter culture; a semi-solid culture method in which culture is performed using a semi-solid medium; or a solid culture method in which culture is performed using a solid medium.

In cases where the filamentous fungus belonging to the genus *Talaromyces* is subjected to liquid culture, potato-dextrose medium or Sabouraud medium may be used therefor. In cases where semi-solid culture is carried out, a medium prepared by gelation of potato-dextrose medium or Sabouraud medium may be used, or a medium prepared by suspending a cereal such as rice; wheat variety; maize; or soybean; or suspending a solid component derived from a cereal, such as bran or soybean meal; in water, may be used. In cases where solid culture is carried out, a medium containing, for example:
a cereal such as rice; wheat variety; maize; or soybean;
a solid component derived from a cereal, such as bran or soybean meal; or
a solid carrier such as a clay mineral containing a nutrient source;

which medium also contains, when necessary, a saccharide, a nitrogen source, or the like,
may be used.

Regarding the culture conditions for the filamentous fungus belonging to the genus *Talaromyces,* the culture is preferably carried out under aerobic conditions by a method such as aeration, stirring, or shaking, and the culture temperature is preferably 20 to 40°C. The culture period is preferably 3 to 60 days, more preferably 3 to 20 days.

The culture containing the filamentous fungus belonging to the genus *Talaromyces* may be used as it is as the disease control agent, or may be, when necessary, homogenized or chopped before use. Alternatively, mainly spores may be collected from the culture by sieving or the like, and the collected product may be used. Alternatively, fungal cells may be separated from the culture using a liquid such as water or oil, and the separated cells may be used as they are or after concentration.

The amount of the filamentous fungus belonging to the genus *Talaromyces* contained in the disease control agent of the present disclosure is not limited, and may be adjusted in accordance with the formulation. For example, in cases of a liquid formulation, the amount is preferably 1×10⁶ to 1×10¹²cfu (colony-forming units)/g, more preferably 1×10⁷ to 1×10¹¹ cfu/g, and, in cases of a solid formulation, the amount is preferably 1×10⁶ to 1×10¹² cfu (colony-forming units)/g, more preferably 1×10⁷ to 1×10¹¹ cfu/g.

As long as the growth, the storage stability, and the disease control effect of the filamentous fungus belonging to the genus *Talaromyces* are not inhibited, the disease control agent of the present disclosure may contain, when necessary, an arbitrary component such as an excipient or a surfactant, for the purpose of formulation, stabilization of the quality, and/or the like. Examples of the arbitrary component used in the disease control agent of the present disclosure include one or more of the following components.

In cases of a solid carrier, examples of the excipient include: fine mineral powders such as kaolin clay, pyrophyllite clay, bentonite, montmorillonite, diatomaceous earth, synthetic hydrated silicon oxide, acid clay, talc, clay, ceramic, quartz, sericite, vermiculite, perlite, Oya stone, anthracite, limestone, stone coal, and zeolite; inorganic compounds such as common salt, carbonates, sulfates, nitrates, and urea; fine organic powders such as chaff, bran, crab shells, shrimp shells, krill powder, rice lees, wheat flour, corn cobs, peanut shells, bone meal, fish meal, lees powder, wood flour, charcoal, kuntan charcoal, bark charcoal, rice husk charcoal, herbal/wooden charcoal, peat moss, attapulgite, dried dung, activated carbon, oil cake, and starch and hydrolysates thereof; and soluble excipients such as D-sorbitol, lactose, maltitose, glucosamine, and oligosaccharides. In cases of a liquid carrier, examples of the excipient include: water, vegetable oils, animal oils, mineral oils, and synthetic water-soluble polymers. One or more of these may be used.

Examples of the surfactant include fatty acid soaps, alkyl ether carboxylic acid, N-acylamino acid, alkyl benzene sulfonate, alkyl naphthalene sulfonate, alpha-olefin sulfonate, dialkyl sulfosuccinate salt, higher alcohol sulfate salt, alkyl ether sulfate, polyoxyethylene alkylphenyl ether sulfate, fatty acid alkylol amide sulfate salt, alkyl ether phosphate salt, alkyl phosphate salt, aliphatic amine salt, aliphatic quaternary ammonium salt, benzalkonium salt, benzethonium chloride, pyridinium salt, imidazolinium salt, polyoxyethylene alkyl ether, polyoxyethylene alkylphenyl ether, polyoxyethylene polyoxypropylene block polymer, polyoxyethylene polyoxypropylene alkyl ether, polyoxyethylene glycerin fatty acid ester, polyoxyethylene castor oil, polyoxyethylene sorbitan fatty acid ester, polyoxyethylene sorbitol fatty acid ester, polyethylene glycol fatty acid ester, fatty acid monoglyceride, polyglycerin fatty acid ester, sorbitan fatty acid ester, fatty acid alkanolamide, polyoxyethylene fatty acid amide, polyoxyethylene alkylamine, alkylamine oxide, carboxybetaine, aminocarboxylate, and imidazolinium betaine. One or more of these may be used.

In cases where a surfactant and an excipient are used, preferably, the filamentous fungus belonging to the genus *Talaromyces* is contained at a ratio of 1 to 90% by mass; the surfactant is contained at a ratio of 1 to 15% by mass; and the excipient is contained at a ratio of 0 to 98% by mass. More preferably, the filamentous fungus belonging to the genus *Talaromyces* is contained at a ratio of 2 to 46% by mass; the surfactant is contained at a ratio of 2 to 15% by mass; and the excipient is contained at a ratio of 39 to 96% by mass.

Since spores of *Talaromyces* fungi are hydrophobic, when they are treated by dilution in water, their dispersibility can be effectively improved by addition of a surfactant. In cases where the amount of the surfactant is too small, the dispersion effect cannot be obtained, while in cases where the amount is too large, the effect reaches the plateau, and the addition is therefore meaningless. Thus, the amount is preferably within the above-described range. By changing the amount of the excipient used within the range, adjustment of the concentration of the effective ingredient is possible.

Further, when necessary, the agent may contain an adjuvant, such as a natural polysaccharide or the like, for example, casein, gelatin, gum arabic, alginic acid, cellulose, carboxymethyl cellulose, xanthan gum, chitin, or chitosan; a polyvinyl alcohol; a polyacrylate; or bentonite; for the purpose of thickening, adhesion, dispersion, and/or the like.

Further, when necessary, the agent may contain a dihydric alcohol or the like, for example, ethylene glycol or propylene glycol, for the purpose of prevention of freezing and/or the like.

Further, when necessary, the agent may contain a surfactant such as an anionic surfactant, a cationic surfactant, or an amphoteric surfactant, for the purpose of dispersion stabilization, aggregation inhibition, emulsification, and/or the like.

The disease control agent of the present disclosure containing a filamentous fungus belonging to the genus *Talaromyces* may be formulated according to an ordinary production method of formulations, into a powder, granule, emulsion, wettable powder, flowable formulation, coating agent, or the like together with an arbitrary component(s) as required.

The wettable powder or the powder may be produced by mixing, or pulverizing and mixing, when necessary, the above-described surfactant and/or a component(s) for stabilization of the quality, with the above-described solid carrier.

The granule may be produced by mixing, or pulverizing and mixing, when necessary, the above-described surfactant and/or a component(s) for stabilization of the quality, with the above-described solid carrier, followed by carrying out granulation.

The emulsion may be produced by mixing, or pulverizing and mixing, when necessary, the above-described surfactant and/or a component(s) for stabilization of the quality, with a liquid carrier such as a vegetable oil, an animal oil, or a mineral oil.

The flowable formulation may be produced by mixing, or pulverizing and mixing, when necessary, the above-described adjuvant, dihydric alcohol and/or the like, surfactant, and/or a component(s) for stabilization of the quality, with water.

The coating agent may be produced by adding an adjuvant to a liquid carrier such as water or oil, and then mixing the resulting mixture into the form of a sol or gel.

### <Target Plants and Target Diseases>

Plants described in this paragraph are for general reference only.

The plant to which the disease control agent of the present disclosure is to be applied is a Brassicaceae plant; bean; maize; wheat variety; tubers and roots; or sugar beet. Examples of the Brassicaceae plant include broccoli, cabbage, cauliflower, kale, turnip, Chinese cabbage (hakusai), Japanese radish (daikon), Japanese horseradish (wasabi), and watercress. Examples of the bean include soybean, pea, common bean, adzuki bean, broad bean, and chickpea. Examples of the wheat variety include wheat, barley, and rye. Examples of the tubers and roots include potato, sweet potato, taro (satoimo), Japanese yam (yamanoimo), and konjac tuber (konnyakuimo).

Diseases and pathogens described in this paragraph are for general reference only.

The target disease is one or more diseases caused by a fungus/fungi selected from the group consisting of fungi belonging to the genus *Rhizoctonia,* fungi belonging to the genus *Fusarium,* fungi belonging to the genus *Pythium,* fungi belonging to the genus *Microdochium,* fungi belonging to the genus *Tilletia,* fungi belonging to the genus *Ustilago,* fungi belonging to the genus *Streptomyces,* fungi belonging to the genus *Erwinia,* and fungi belonging to the genus *Aphanomyces.*

Examples of the fungi belonging to the genus *Rhizoctonia* include *Rhizoctonia solani,* and examples of the diseases caused by the fungi belonging to the genus *Rhizoctonia* include damping-off.

Examples of the fungi belonging to the genus *Fusarium* include *Fusarium solani* and *Fusarium oxysporum,* and examples of the diseases caused by the fungi belonging to the genus *Fusarium* include damping-off.

Examples of the fungi belonging to the genus *Pythium* include *Pythium ultimum, Pythium aphanidermatum, Pythium spinosum, Pythium debaryanum, Pythium cucurbitacearum,* and *Pythium myriotylum,* and examples of the diseases caused by the fungi belonging to the genus *Pythium* include damping-off.

Examples of the fungi belonging to the genus *Microdochium* include *Microdochium nival,* and examples of the diseases caused by the fungi belonging to the genus *Microdochium* include pink snow mold.

Examples of the fungi belonging to the genus *Tilletia* include *Tilletia caries, Tilletia tritici, Tilletia leavis,* and *Tilletia foetida,* and examples of the diseases caused by the fungi belonging to the genus *Tilletia* include stinking smut.

Examples of the fungi belonging to the genus *Ustilago* include *Ustilago nuda,* and examples of the diseases caused by the fungi belonging to the genus *Ustilago* include loose smut.

Examples of the fungi belonging to the genus *Streptomyces* include *Streptomyces scabiei,* and examples of the diseases caused by the fungi belonging to the genus *Streptomyces* include scab.

Examples of the fungi belonging to the genus *Erwinia* include *Erwinia carotovora* and *Erwinia chrysanthemi,* and examples of the diseases caused by the fungi belonging to the genus *Erwinia* include blackleg.

Examples of the fungi belonging to the genus *Aphanomyces* include *Aphanomyces cochlioides,* and examples of the diseases caused by the fungi belonging to the genus *Aphanomyces* include damping-off and black root rot.

Combinations of plant and diseases or pathogens described in this paragraph are for reference only. Combinations to be treated by the use of *Talaromyces flavus* Y-9401 are defined in the appended claim 1 and in the foregoing paragraph [0015].

In cases where the disease control agent of the present disclosure is applied to Brassicaceae plant, the agent has an excellent control effect especially against diseases caused by fungi belonging to the genus *Rhizoctonia,* fungi belonging to the genus *Fusarium,* and fungi belonging to the genus *Pythium.*

In cases where the disease control agent of the present disclosure is applied to beans such as soybean, the agent has an excellent control effect especially against diseases caused by fungi belonging to the genus *Rhizoctonia,* fungi belonging to the genus *Fusarium,* and fungi belonging to the genus *Pythium.*

In cases where the disease control agent of the present disclosure is applied to maize, the agent has an excellent control effect especially against diseases caused by fungi belonging to the genus *Rhizoctonia,* fungi belonging to the genus *Fusarium,* and fungi belonging to the genus *Pythium.*

In cases where the disease control agent of the present disclosure is applied to wheat variety, the agent has an excellent control effect especially against diseases caused by fungi belonging to the genus *Ustilago,* fungi belonging to the genus *Tilletia,* fungi belonging to the genus *Microdochium,* fungi belonging to the genus *Rhizoctonia,* fungi belonging to the genus *Fusarium,* and fungi belonging to the genus *Pythium.*

In cases where the disease control agent of the present disclosure is applied to tubers and roots such as potato, the agent has an excellent control effect especially against diseases caused by fungi belonging to the genus *Streptomyces,* fungi belonging to the genus *Erwinia,* and fungi belonging to the genus *Rhizoctonia.*

In cases where the disease control agent of the present disclosure is applied to sugar beet, the agent has an excellent control effect especially against diseases caused by fungi belonging to the genus *Aphanomyces.*

### <Application Method>

The disease control agent of the present disclosure containing a filamentous fungus belonging to the genus *Talaromyces* is applied for treatment of seeds, seedlings, plant bodies (leaves, stems, roots, and the like), seedling culture soil, seedling culture medium, cultivation soil, cultivation medium, and/or the like of a Brassicaceae plant; bean; maize; wheat variety; tubers and roots; or sugar beet; for the purpose of controlling the diseases described above. The method of the application is appropriately selected in accordance with the mode of use such as the formulation, and the disease.

Examples of the method include seed soaking treatment, seed dressing treatment, seed coating treatment, spraying treatment onto seeds, spraying treatment onto soil, soil incorporation application, soil drenching application, seedling-tray drenching application, base application, liquid spraying onto the above-ground part, solid spraying onto the above-ground part, and the like. These may be carried out repeatedly or in combination.

The amount of the disease control agent of the present disclosure containing a filamentous fungus belonging to the genus *Talaromyces* to be applied may vary depending on the type of the disease and the like. For example, in cases where seed soaking treatment is carried out, the agent is preferably applied as a seed soaking liquid in which the preparation is diluted 10- to 1000-fold (by mass), and the fungal concentration is usually 1×10³ to 1×10¹⁰ cfu, preferably 1×10⁴ to 1×10⁹ cfu per 1 ml of the soaking liquid.

In cases of seed dressing treatment, the preparation is preferably applied at 1 to 20% by mass with respect to the seed mass. The fungal concentration is usually 1×10³ to 1×10¹⁰ cfu, preferably 1×10⁴ to 1×10⁹ cfu per seed mass of 1 g.

In cases of spraying treatment onto soil, when a liquid is sprayed onto a seedling tray (with an area of, for example, about 1800 cm²), the agent is preferably applied in an amount of 50 to 1000 ml, and the fungal concentration is usually 1×10³ to 1×10¹⁰ cfu, preferably 1×10⁴ to 1×10¹⁰ cfu per 1 ml of of the spray liquid. Also in cases where a material other than a seedling tray is used for the seedling culture or cultivation, the same amount of the agent may be applied per unit area.

In cases of soil incorporation application, the agent is preferably applied in an amount of 0.1 to 100 g per seedling tray (with an area of, for example, about 1800 cm²), and the fungal concentration is usually 1×10² to 1×10⁹ cfu, preferably 1×10³ to 1×10⁸ cfu per 1 ml of of the soil. Also in cases where a material other than a seedling tray is used for the seedling culture or cultivation, the same amount of the agent may be applied per unit area.

In cases of drenching application to the soil, when a liquid is sprayed onto a seedling tray (with an area of, for example, about 1800 cm²), the agent is preferably applied in an amount of 50 to 1000 ml, and the fungal concentration is usually 1×10³ to 1×10⁹ cfu, preferably 1×10⁴ to 1×10⁸ cfu per 1 ml of of the spray liquid. Also in cases where a material other than a seedling tray is used for the seedling culture or cultivation, the same amount of the agent may be applied per unit area.

As long as the growth and the controlling effect of the filamentous fungus belonging to the genus *Talaromyces* are not adversely affected, the control agent of the present disclosure may be used as a mixture with, or in combination with, other disease control agents. For example, the agent may be used in combination with a microbicide, an insecticide, a nematicide, a miticide, an herbicide, a plant growth regulator, a fertilizer, and/or a microbial agent.

### EXAMPLES

The present invention is described below in more detail by way of Examples.

Test on Control Effect against Cabbage Damping-Off Caused by *Rhizoctonia*

### <Production Example 1>

### (Production of Spores)

Wheat bran was used as a medium. An inoculum of the *Talaromyces flavus* Y-9401 strain was inoculated to the medium, and solid culture was carried out at 30°C for 10 days. After completion of the culture, the culture was dried to obtain a powder containing spores of the *Talaromyces flavus* Y-9401 strain (4×10⁹ cfu/g).

### (Production of Formulation)

The powder containing spores of the *Talaromyces flavus* Y-9401 strain obtained above in Production Example 1 was used. A mixture containing this spore-containing powder at 10% by mass, SORPOL4315L (manufactured by TOHO Chemical Industry Co., Ltd.) as a surfactant at 10% by mass, and soybean oil (manufactured by Nisshin Oillio Group, Ltd.) as an excipient at 80% by mass was prepared, and the mixture was stirred and filtered through a 180-µm sieve. The resulting filtrate was concentrated using a centrifuge, to obtain a flowable formulation (1×10⁹ cfu/g).

### <Example 1>

### (Test on Control Effect against Cabbage Damping-Off Caused by Rhizoctonia)

### (1) Inoculation of Pathogenic Fungus

A horticulture soil (Genkikun No. 1) containing a fertilizer component was mixed with *Rhizoctonia solani* fungi obtained by static culture in potato dextrose broth (PDB) medium, such that the fungi were contained at 1/50 by weight, to provide a Rhizoctonia-contaminated cultivation soil.

### (2) Treatment with Agent

Twenty seeds of cabbage (variety: Okina) were sown in a plastic cup with a diameter of 6.5 cm filled with the Rhizoctonia-contaminated cultivation soil. The flowable formulation prepared in the above production of the preparation was diluted to 1×10⁷ cfu/ml, 4×10⁶ cfu/ml, or 2×10⁶ cfu/ml with water, and uniformly sprayed (9.3 ml) onto the surface of the soil, to provide agent treatment groups. In addition, a wettable powder (Rizolex wettable powder) containing tolclofos-methyl as an effective ingredient was diluted 500-fold with water, and uniformly sprayed (9.3 ml) onto the surface of the soil, to provide an agent treatment control group. In addition, a pot was prepared using water instead of the agent solution, to provide a control (untreated group). Three independent tests were carried out for both the agent treatment groups and the untreated group.

### (3) Cultivation of Test Plants

Each plastic cup subjected to the above treatment was covered with the *Rhizoctonia-contaminated* cultivation soil, and then cultivation management was carried out in a glasshouse according to a conventional method.

### (4) Investigation of Control Effect

One week after the sowing, seeds showing no budding and seedlings exhibiting symptoms of damping-off caused by *Rhizoctonia* fungi, such as rot or wilt, were regarded as diseased seedlings, and the rate of healthy seedlings in each treatment group was calculated.

### Results

It was found, as shown in Table 1, that damping-off caused by *Rhizoctonia solani* can be controlled by soil drenching treatment with the *Talaromyces* agent.

**[Table 1]**

| | | **Untreated inoculation group** | **Untreated non-inoculation group** | 1×10⁷ cfu/ml | 4×10⁶ cfu/ml | 2×10⁶ cfu/ml | **Rizolex** ×500 |
|---|---|---|---|---|---|---|---|
| **Number of seeds** | | 20 | 20 | 20 | 20 | 20 | 20 |
| **Number of healthy seedlings** | I | 15 | 20 | 19 | 18 | 18 | 19 |
| | II | 9 | 18 | 18 | 19 | 17 | 18 |
| | III | 7 | 20 | 18 | 17 | 15 | 20 |
| **Average** | | 10.3 | 19.3 | 18.3 | 18.0 | 16.7 | 19.0 |
| **Rate of healthy seedlings** | | 51.7% | 96.7% | 91.7% | 90.0% | 83.3% | 95.0% |

### <Production Example 2>

### (Production of Spores)

Wheat bran was used as a medium. An inoculum of the *Talaromyces flavus* Y-9401 strain was inoculated to the medium, and solid culture was carried out at 30°C for 10 days. After completion of the culture, the culture was dried, and a mixture of a clay mineral (Kaolin KH, Kanaya Kosan) as an excipient and the dried culture, prepared at a weight ratio of 1:9, was subjected to sieving to remove bran residues, to obtain a powder containing spores of the *Talaromyces flavus* Y-9401 strain (1×10¹⁰ cfu/g).

### <Formulation Example 2>

### (Production of Formulation)

The powder containing spores of *Talaromyces flavus* Y-9401 obtained above in Production Example 2 was used. A mixture containing this spore-containing powder at 20% by mass, SORPOL5082 (manufactured by TOHO Chemical Industry Co., Ltd.) as a surfactant at 10% by mass, clay mineral (Kaolin KH) as an excipient at 38% by mass, glucosamine (manufactured by Protein Chemical Co., Ltd.) at 30% by mass, and Sumecton SA (manufactured by Kunimine Industries Co., Ltd.) as a thickener at 2 % by mass was prepared, and the mixture was mixed and pulverized using a grinder mill, to obtain a wettable powder of Formulation Example 2 (2×10⁹ cfu/g).

### <Production Example 3>

### (Production of Spores)

Wheat bran was used as a medium. An inoculum of the *Talaromyces flavus* Y-9401 strain was inoculated to the medium, and solid culture was carried out at 30°C for 10 days. After completion of the culture, the culture was dried, and the dried culture was subjected to sieving to remove wheat bran residues, to obtain a powder containing spores of *Talaromyces flavus* Y-9401 (4×10⁹ cfu/g).

### <Formulation Example 3>

### (Production of Formulation)

The powder containing spores of *Talaromyces flavus* Y-9401 obtained above in Production Example 3 was used. A mixture containing this spore-containing powder at 10% by mass, SORPOL5082 (manufactured by TOHO Chemical Industry Co., Ltd.) as a surfactant at 5% by mass, clay mineral (HA-A Kaolin Clay, manufactured by Maruo Calcium Co., Ltd.) as an excipient at 45% by mass, and glucosamine (manufactured by Protein Chemical Co., Ltd.) at 40% by mass was prepared, and the mixture was mixed and pulverized using a grinder mill, to obtain a wettable powder of Formulation Example 3 (4×10⁸ cfu/g).

### <Example 2>

### (Test on Control Effect against Wheat Damping-Off Caused by Pythium)

### (1) Inoculation of Pathogenic Fungus

*Pythium ultimum* were subjected to shake culture in a cornmeal liquid medium. A medium was prepared by mixing soil and bran together at a volume ratio of 2:1, and adjusting the water content to 50%, followed by sterilization by autoclaving. The cultured pathogen were inoculated to the prepared medium, and solid culture was carried out for seven days. The resulting culture was mixed at a volume ratio of 1:4 with soil sterilized by autoclaving, to provide a contaminated soil.

### (2) Treatment with Agent

Ten seeds of wheat (variety: Norin No. 61) were sown in a plastic cup with a diameter of 5.5 cm filled with the soil contaminated with *Pythium ultimum.* The sown seeds were completely covered with soil. The wettable powder prepared in Formulation Example 2 was diluted to 2× 10⁸ cfu/ml, 7×10⁷ cfu/ml, or 2×10⁷ cfu/ml with water, and uniformly sprayed (15 ml) onto the surface of the soil, to provide agent treatment groups. In addition, for comparison, wheat seeds were subjected to dressing treatment at 0.4% by weight with a wettable powder (Orthocide wettable powder 80) containing Captan as an effective ingredient, and then sown to provide an agent treatment control group. In addition, an untreated inoculation group, in which no treatment with the agent solution was carried out at all, and an untreated non-inoculation group, in which 10 wheat seeds were sown in sterilized healthy soil, were provided. For all of the test groups, three independent tests were carried out.

### (3) Cultivation of Test Plants

Each plastic cup subjected to the above treatment was placed in a greenhouse, and cultivation management was carried out according to a conventional method.

### (4) Investigation of Control Effect

Two weeks after the sowing, seeds showing no budding and seedlings exhibiting symptoms of damping-off caused by *Pythium,* such as rot or wilt, were regarded as diseased seedlings, and the rate of healthy seedlings in each treatment group was calculated.

### Results

It was found, as shown in Table 2, that damping-off caused by *Pythium ultimum* can be controlled by treatment with the *Talaromyces* agent.

**[Table 2]**

| | | **Untreated inoculation group** | **Untreated non-inoculation group** | 2×10⁸ cfu/ml | 7×10⁷ cfu/ml | 2×10⁷ cfu/ml | **Orthocide** 0.4% |
|---|---|---|---|---|---|---|---|
| **Number of seeds** | | 10 | 10 | 10 | 10 | 10 | 10 |
| **Number of healthy seedlings** | I | 1 | 10 | 9 | 9 | 7 | 10 |
| | II | 0 | 9 | 10 | 8 | 7 | 6 |
| | III | 4 | 10 | 10 | 10 | 9 | 8 |
| **Average** | | 1.7 | 9.7 | 9.7 | 9.0 | 7.7 | 8.0 |
| **Rate of healthy seedlings** | | 16.7% | 96.7% | 96.7% | 90.0% | 76.7% | 80.0% |

### <Example 3>

### (Test on Control Effect against Dent Corn Damping-Off Caused by Pythium)

### (1) Inoculation of Pathogenic Fungus

The culture prepared in Example 2 was mixed at a volume ratio of 1:9 with soil sterilized by autoclaving, to provide a contaminated soil.

### (2) Treatment with Agent

Five seeds of dent corn (variety: 34N84) were sown in a plastic cup with a diameter of 5.5 cm filled with the soil contaminated with *Pythium ultimum.* The sown seeds were completely covered with soil. The wettable powder prepared in Formulation Example 2 was diluted to 2×10⁸ cfu/ml, 7×10⁷ cfu/ml, or 2×10⁷ cfu/ml with water, and uniformly sprayed (15 ml) onto the surface of the soil, to provide agent treatment groups. In addition, for comparison, dent corn seeds were subjected to dressing treatment at 0.4% by weight with a wettable powder (Orthocide wettable powder 80) containing Captan as an effective ingredient, and then sown to provide an agent treatment control group. In addition, an untreated inoculation group, in which no treatment with the agent solution was carried out at all, and an untreated non-inoculation group, in which five dent corn seeds were sown in sterilized healthy soil, were provided. For all of the test groups, three independent tests were carried out.

### (3) Cultivation of Test Plants

Each plastic cup subjected to the above treatment was placed in a greenhouse, and cultivation management was carried out according to a conventional method.

### (4) Investigation of Control Effect

Two weeks after the sowing, seeds showing no budding and seedlings exhibiting symptoms of damping-off caused by *Pythium,* such as rot or wilt, were regarded as diseased seedlings, and the rate of healthy seedlings in each treatment group was calculated.

### Results

It was found, as shown in Table 3, that damping-off caused by *Pythium ultimum* can be controlled by treatment with the *Talaromyces* agent.

**[Table 3]**

| | | **Untreated inoculation group** | **Untreated non-inoculation group** | 2×10⁸ cfu/ml | 7×10⁷ cfu/ml | 2×10⁷ cfu/ml | **Orthocide** 0.4% |
|---|---|---|---|---|---|---|---|
| **Number of seeds** | | 5 | 5 | 5 | 5 | 5 | 5 |
| **Number of healthy seedlings** | I | 0 | 4 | 2 | 1 | 1 | 5 |
| | II | 0 | 4 | 4 | 2 | 0 | 1 |
| | III | 0 | 5 | 1 | 2 | 0 | 2 |
| **Average** | | 0.0 | 4.3 | 2.3 | 1.7 | 0.3 | 2.7 |
| **Rate of healthy seedlings** | | 0.0% | 86.7% | 46.7% | 33.3% | 6.7% | 53.3% |

### <Example 4>

### (Test on Control Effect against Soybean Damping-Off Caused by Fusarium)

### (1) Inoculation of Pathogenic Fungus

*Fusarium oxysporum* fungi were subjected to static culture on potato dextrose agar medium (PDA medium). A medium was prepared by mixing soil and cornmeal together at a volume ratio of 5:1, and adjusting the water content to 50%, followed by sterilization by autoclaving. The cultured pathogens were inoculated to the prepared medium, and solid culture was carried out for 21 days. The resulting culture was mixed at a volume ratio of 1:4 with soil sterilized by autoclaving, to provide a contaminated soil.

### (2) Treatment with Agent

Five seeds of soybean (variety: Enrei) were sown in a plastic cup with a diameter of 5.5 cm filled with the soil contaminated with *Fusarium oxysporum.* The sown seeds were completely covered with soil. The wettable powder prepared in Formulation Example 2 was diluted to 2×10⁸ cfu/ml or 2×10⁷ cfu/ml with water, and uniformly sprayed (15 ml) onto the surface of the soil, to provide agent treatment groups. In addition, for comparison, soybean seeds were subjected to dressing treatment at 0.5% by weight with a wettable powder (Homai wettable powder) containing thiuram and thiophanate-methyl as effective ingredients, and then sown to provide an agent treatment control group. In addition, an untreated inoculation group, in which no treatment with the agent solution was carried out at all, and an untreated non-inoculation group, in which five soybean seeds were sown in sterilized healthy soil, were provided. For all of the test groups, three independent tests were carried out.

### (3) Cultivation of Test Plants

Each plastic cup subjected to the above treatment was placed in a greenhouse, and cultivation management was carried out according to a conventional method.

### (4) Investigation of Control Effect

Two weeks after the sowing, seeds showing no budding and seedlings exhibiting symptoms of damping-off caused by *Fusarium,* such as rot or wilt, were regarded as diseased seedlings, and the rate of healthy seedlings in each treatment group was calculated.

### Results

It was found, as shown in Table 4, that damping-off caused by *Fusarium oxysporum* can be controlled by soil drenching treatment with the *Talaromyces* agent.

**[Table 4]**

| | | **Untreated inoculation group** | **Untreated non-inoculation group** | 2×10⁸ cfu/ml | 2×10⁷ cfu/ml | **Homai** 0.5% |
|---|---|---|---|---|---|---|
| **Number of seeds** | | 5 | 5 | 5 | 5 | 5 |
| **Number of healthy seedlings** | I | 0 | 4 | 1 | 3 | 2 |
| | II | 1 | 3 | 3 | 2 | 1 |
| | III | 1 | 4 | 3 | 1 | 0 |
| **Average** | | 0.7 | 3.7 | 2.3 | 2.0 | 1.0 |
| **Rate of healthy seedlings** | | 13.3% | 73.3% | 46.7% | 40.0% | 20.0% |

### <Example 5>

### (Test on Control Effect against Potato Scab)

### (1) Treatment with Agent

An agent liquid was prepared by diluting the wettable powder prepared above in Formulation Example 3, to 1×10⁶ cfu/ml. Contaminated potatoes (variety: Nishiyutaka) infected with *Streptomyces scabiei,* having about 4 to 10 lesions per seed potato, were soaked for 1 minute or 10 seconds in the prepared agent liquid. After drying the agent liquid, the seed potatoes were sown in a field. In addition, for comparison, an agent liquid was prepared by diluting 100-fold a wettable powder (Agrimycin wettable powder 100) containing as effective ingredients streptomycin and oxytetracycline, and the potatoes were soaked therein for 10 seconds, followed by drying, to provide an agent treatment control group. In addition, an untreated inoculation group was provided by soaking only in water followed by drying. For all of the test groups, three independent tests were carried out.

### (2) Investigation of Control Effect

The potatoes were harvested 81 days after the sowing. Those exhibiting lesions of scab on the stem-tuber surface were regarded as diseased stem tubers, and the rate of diseased stem tubers with respect to the total number of stem tubers was calculated.

### Results

It was found, as shown in Table 5, that scab caused by *Streptomyces scabiei* can be controlled by treatment with the *Talaromyces* agent.

**[Table 5]**

| No. | **Treatment** | **Yield of diseased stem tubers** | **Total number of stem tubers** | **Rate of diseased stem tubers** |
|---|---|---|---|---|
| | | **Number** | **Number** | |
| I | 1×10⁶cfu/ml **1-minute soaking** | 75 | 126 | 59.5% |
| II | | 53 | 105 | 50.5% |
| III | | 56 | 119 | 47.1% |
| **Average** | | 61.3 | 116.7 | 52.4% |
| I | 1×10⁶cfu/ml **10-second soaking** | 45 | 102 | 44.1% |
| II | | 40 | 82 | 48.8% |
| III | | 35 | 96 | 36.5% |
| **Average** | | 40.0 | 93.3 | 43.1% |
| I | **Agrimycin 100× 10-second soaking** | 44 | 79 | 55.7% |
| II | | 25 | 64 | 39.1% |
| III | | 37 | 82 | 45.1% |
| **Average** | | 35.3 | 75.0 | 46.6% |
| I | **Untreated inoculation group** | 87 | 98 | 88.8% |
| II | | 90 | 124 | 72.6% |
| III | | 97 | 132 | 73.5% |
| **Average** | | 91.3 | 118.0 | 78.3% |

## Claims

1. Use of an agent comprising a filamentous fungus belonging to the genus *Talaromyces* for controlling a disease for a plant selected from the group consisting of cabbage; soybeans; maize; wheat variety; and potato, wherein
when said plant is cabbage said disease is caused by fungi belonging to the genus *Rhizoctonia,*
when said plant is maize said disease is caused by fungi belonging to the genus *Pythium,*
when said plant is wheat variety said disease is caused by fungi belonging to the genus *Pythium,*
when said plant is soybean said disease is caused by fungi belonging to the genus *Fusarium,* and
when said plant is potato said disease is caused by bacteria belonging to the genus *Streptomyces* or fungi belonging to the genus *Rhizoctonia,* and
wherein the filamentous fungus belonging to the genus *Talaromyces* is *Talaromyces flavus* and wherein the *Talaromyces flavus* is *Talaromyces flavus* Y-9401 strain (FERM BP-10642).

2. The use according to claim 1, wherein said agent comprises the *Talaromyces flavus Y-*9401 strain (FERM BP-10642) in an amount of 1×10⁶ to 1×10¹² colony-forming units/g.

3. The use according to any one of claims 1 to 2, wherein said agent further comprising a surfactant and/or an excipient.

4. The use according to claim 3, wherein the surfactant is one or more surfactants selected from the group consisting of fatty acid soaps, alkyl ether carboxylic acid, N-acylamino acid, alkyl benzene sulfonate, alkyl naphthalene sulfonate, alpha-olefin sulfonate, dialkyl sulfosuccinate salt, higher alcohol sulfate salt, alkyl ether sulfate, polyoxyethylene alkylphenyl ether sulfate, fatty acid alkylol amide sulfate salt, alkyl ether phosphate salt, alkyl phosphate salt, aliphatic amine salt, aliphatic quaternary ammonium salt, benzalkonium salt, benzethonium chloride, pyridinium salt, imidazolinium salt, polyoxyethylene alkyl ether, polyoxyethylene alkylphenyl ether, polyoxyethylene polyoxypropylene block polymer, polyoxyethylene polyoxypropylene alkyl ether, polyoxyethylene glycerin fatty acid ester, polyoxyethylene castor oil, polyoxyethylene sorbitan fatty acid ester, polyoxyethylene sorbitol fatty acid ester, polyethylene glycol fatty acid ester, fatty acid monoglyceride, polyglycerin fatty acid ester, sorbitan fatty acid ester, fatty acid alkanolamide, polyoxyethylene fatty acid amide, polyoxyethylene alkylamine, alkylamine oxide, carboxybetaine, aminocarboxylate, and imidazolinium betaine.

5. The use according to claim 3 or 4, wherein the excipient is one or more excipients selected from the group consisting of:
fine mineral powders selected from the group consisting of kaolin clay, pyrophyllite clay, bentonite, montmorillonite, diatomaceous earth, synthetic hydrated silicon oxide, acid clay, talc, clay, ceramic, quartz, sericite, vermiculite, perlite, Oya stone, anthracite, limestone, stone coal, and zeolite;
inorganic compounds selected from the group consisting of common salt, carbonates, sulfates, nitrates, and urea;
organic matters selected from the group consisting of chaff, bran, crab shells, shrimp shells, krill powder, rice lees, wheat flour, corn cobs, peanut shells, bone meal, fish meal, lees powder, wood flour, charcoal, kuntan charcoal, bark charcoal, rice husk charcoal, herbal/wooden charcoal, peat moss, attapulgite, dried dung, activated carbon, oil cake, and starch and hydrolysates thereof;
saccharides selected from the group consisting of D-sorbitol, lactose, maltitose, glucosamine, and oligosaccharides;
oils selected from the group consisting of vegetable oils, animal oils, and mineral oils; and
synthetic water-soluble polymers.

6. The use according to any one of claims 3 to 5, wherein said agent comprises the *Talaromyces flavus* Y-9401 strain (FERM BP-10642) at 1 to 90% by mass, the surfactant at 1 to 15% by mass, and the excipient at 0 to 98% by mass.

7. The use according to any one of claims 3 to 5, wherein said agent comprises *Talaromyces flavus* Y-9401 strain (FERM BP-10642) at 2 to 46% by mass, the surfactant at 2 to 15% by mass, and the excipient at 39 to 96% by mass.

8. The use according to any one of claims 1 to 7, wherein said agent is formulated as any of a powder, a granule, an emulsion, a wettable powder, a flowable formulation, and a coating agent.

9. A method of controlling a disease of a plant, the method comprising:
treating seeds, seedlings, plant bodies, seedling culture soil, seedling culture medium, cultivation soil, and/or cultivation medium of the plant using an agent comprising *Talaromyces flavus* Y-9401 strain (FERM BP-10642);
wherein the plant is selected from the group consisting of cabbage; soybeans; maize;
wheat variety; and potato; and
when said plant is cabbage said disease is caused by fungi belonging to the genus *Rhizoctonia,*
when said plant is maize said disease is caused by fungi belonging to the genus *Pythium,*
when said plant is wheat variety said disease is caused by fungi belonging to the genus *Pythium,*
when said plant is soybean said disease is caused by fungi belonging to the genus *Fusarium,* and
when said plant is potato said disease is caused by bacteria belonging to the genus *Streptomyces* or fungi belonging to the genus *Rhizoctonia.*

## Patentansprüche

1. Verwendung eines Mittels, das einen fadenförmigen Pilz der Gattung *Talaromyces* umfasst, zur Bekämpfung einer Krankheit bei einer Pflanze, die aus der Gruppe bestehend aus Kohl, Sojabohnen, Mais, Weizensorten und Kartoffeln ausgewählt ist, wobei,
wenn die Pflanze Kohl ist, die Krankheit durch Pilze der Gattung *Rhizoctonia* verursacht wird,
wenn die Pflanze Mais ist, die Krankheit durch Pilze der Gattung *Pythium* verursacht wird,
wenn die Pflanze eine Weizensorte ist, die Krankheit durch Pilze der Gattung *Pythium* verursacht wird,
wenn die Pflanze Sojabohne ist, die Krankheit durch Pilze der Gattung *Fusarium* verursacht wird, und
wenn die Pflanze Kartoffel ist, die Krankheit durch Bakterien der Gattung *Streptomyces* oder Pilze der Gattung *Rhizoctonia* verursacht wird, und
wobei der fadenförmige Pilz der Gattung *Talaromyces Talaromyces flavus* ist und wobei der *Talaromyces flavus* der Stamm *Talaromyces flavus* Y-9401 (FERM BP-10642) ist.

2. Verwendung nach Anspruch 1, wobei das Mittel den Stamm *Talaromyces flavus* Y-9401 (FERM BP-10642) in einer Menge von 1×10⁶ bis 1×10¹² koloniebildenden Einheiten/g umfasst.

3. Verwendung nach einem der Ansprüche 1 bis 2, wobei das Mittel weiterhin eine grenzflächenaktive Substanz und/oder einen Hilfsstoff umfasst.

4. Verwendung nach Anspruch 3, wobei die grenzflächenaktive Substanz ein oder mehrere grenzflächenaktive Substanzen ist, ausgewählt aus der Gruppe bestehend aus Fettsäureseifen, Alkylethercarbonsäure, N-Acylaminosäure, Alkylbenzolsulfonat, Alkylnaphthalinsulfonat, Alpha-Olefinsulfonat, Dialkylsulfosuccinatsalz, höherem Alkoholsulfatsalz, Alkylethersulfat, Polyoxyethylen-alkylphenylethersulfat, Fettsäurealkylolamidsulfatsalz, Alkyletherphosphatsalz, Alkyl-phosphatsalz, aliphatisches Aminsalz, aliphatisches quaternäres Ammoniumsalz, Benzalkoniumsalz, Benzethoniumchlorid, Pyridiniumsalz, Imidazoliniumsalz, Polyoxy-ethylenalkylether, Polyoxyethylenalkylphenylether, Polyoxyethylen-Polyoxypropylen-Blockcopolymer, Polyoxyethylen-Polyoxypropylen-Alkylether, Polyoxyethylenglycerin-Fettsäureester, Polyoxyethylen-Rizinusöl, Polyoxyethylensorbitan-Fettsäureester, Polyoxyethylensorbitol-Fettsäureester, Polyethylenglykol-Fettsäureester, Fettsäuremonoglycerid, Polyglycerin-Fettsäureester, Sorbitan-Fettsäureester, Fettsäurealkanolamid, Polyoxyethylen-Fettsäureamid, Polyoxyethylen-Alkylamin, Alkylaminoxid, Carboxybetain, Aminocarboxylat und Imidazoliniumbetain.

5. Verwendung nach Anspruch 3 oder 4, wobei der Hilfsstoff ein oder mehrere Hilfsstoffe ist, ausgewählt aus der Gruppe bestehend aus:
feinen Mineralpulvern, ausgewählt aus der Gruppe bestehend aus Kaolin-Ton, Pyrophyllit-Ton, Bentonit, Montmorillonit, Kieselgur, synthetischem hydratisiertem Siliziumoxid, saurem Ton, Talk, Ton, Keramik, Quarz, Serizit, Vermiculit, Perlit, Oya-Stein, Anthrazit, Kalkstein, Steinkohle und Zeolith;
anorganischen Verbindungen, ausgewählt aus der Gruppe bestehend aus Kochsalz, Carbonaten, Sulfaten, Nitraten und Harnstoff;
organischen Stoffen, ausgewählt aus der Gruppe bestehend aus Spreu, Kleie, Krabbenpanzern, Garnelenschalen, Krillpulver, Reishefe, Weizenmehl, Maiskolben, Erdnussschalen, Knochenmehl, Fischmehl, Hefepulver, Holzmehl, Holzkohle, Kuntan-Holzkohle, Rindenholzkohle, Reisschalen-Holzkohle, Kräuter-/Holz-Kohle, Torfmoos, Attapulgit, getrocknetem Dung, Aktivkohle, Ölkuchen sowie Stärke und deren Hydrolysate;
Sacchariden ausgewählt aus der Gruppe bestehend aus d-Sorbitol, Laktose, Maltitose, Glucosamin und Oligosacchariden;
Ölen ausgewählt aus der Gruppe bestehend aus Pflanzenölen, tierischen Ölen und Mineralölen; und
synthetischen wasserlöslichen Polymeren.

6. Verwendung nach einem der Ansprüche 3 bis 5, wobei das Mittel den Stamm *Talaromyces flavus* Y-9401 (FERM BP-10642) zu 1 bis 90 Massenprozent, die grenzflächenaktive Substanz zu 1 bis 15 Massenprozent und den Hilfsstoff zu 0 bis 98% Massenprozent umfasst.

7. Verwendung nach einem der Ansprüche 3 bis 5, wobei das Mittel den Stamm *Talaromyces flavus* Y-9401 (FERM BP-10642) zu 2 bis 46 Massenprozent, die grenzflächenaktive Substanz zu 2 bis 15 Massenprozent und den Hilfsstoff zu 39 bis 96 Massenprozent umfasst.

8. Verwendung nach einem der Ansprüche 1 bis 7, wobei das Mittel in Form eines Pulvers, eines Granulats, einer Emulsion, eines benetzbaren Pulvers, einer fließfähigen Formulierung oder eines Beschichtungsmittels formuliert ist.

9. Verfahren zur Bekämpfung einer Krankheit einer Pflanze, wobei das Verfahren umfasst:
Behandlung von Samen, Sämlingen, Pflanzenbestandteilen, Anzuchterde, Anzuchtmedium, Kulturerde und/oder Kultivierungsmedium der Pflanze mit einem Mittel, das den Stamm *Talaromyces flavus* Y-9401 (FERM BP-10642) umfasst;
wobei die Pflanze aus der Gruppe bestehend aus Kohl, Sojabohnen, Mais, Weizensorten und Kartoffeln ausgewählt ist; und
wenn die Pflanze Kohl ist, die Krankheit durch Pilze der Gattung *Rhizoctonia* verursacht wird,
wenn Pflanze Mais ist, die Krankheit durch Pilze der Gattung *Pythium* verursacht wird,
wenn Pflanze eine Weizensorte ist, die Krankheit durch Pilze der Gattung *Pythium* verursacht wird,
wenn Pflanze eine Sojabohne ist, die Krankheit durch Pilze der Gattung *Fusarium* verursacht wird, und
wenn die Pflanze Kartoffel ist, die Krankheit durch Bakterien der Gattung *Streptomyces* oder Pilze der Gattung *Rhizoctonia* verursacht wird.

## Revendications

1. Utilisation d'un agent comprenant un champignon filamenteux appartenant au genre *Talaromyces* pour lutter contre une maladie d'une plante choisie dans le groupe constitué par le chou; le soja; le maïs; les variétés de blé; et la pomme de terre, dans laquelle
quand ladite plante est un chou, ladite maladie est provoquée par des champignons appartenant au genre *Rhizoctonia,*
quand ladite plante est le maïs, ladite maladie est provoquée par des champignons appartenant au genre *Pythium,*
quand ladite plante est une variété de blé, ladite maladie est provoquée par des champignons appartenant au genre *Pythium,*
quand ladite plante est le soja, ladite maladie est provoquée par des champignons appartenant au genre *Fusarium,* et
quand ladite plante est la pomme de terre, ladite maladie est provoquée par des bactéries appartenant au genre *Streptomyces* ou des champignons appartenant au genre *Rhizoctonia,* et
dans laquelle le champignon filamenteux appartenant au genre *Talaromyces* est *Talaromyces flavus* et dans laquelle le *Talaromyces flavus* est la souche de *Talaromyces flavus* Y-9401 (FERM BP-10642).

2. Utilisation selon la revendication 1, dans laquelle ledit agent comprend la souche de *Talaromyces flavus* Y-9401 (FERM BP-10642) en une quantité de 1 × 10⁶ à 1 × 10¹² unités formant colonies par gramme.

3. Utilisation selon l'une quelconque des revendications 1 à 2, dans laquelle ledit agent comprend en outre un tensioactif et/ou un excipient.

4. Utilisation selon la revendication 3, dans laquelle le tensioactif est un ou plusieurs tensioactifs choisis dans le groupe constitué par les savons d'acides gras, un acide alkyléthercarboxylique, un acide N-acylaminé, un alkylbenzènesulfonate, un alkylnaphtalènesulfonate, une alpha-oléfinesulfonate, un sel dialkylsulfosuccinate, un sel sulfate d'alcool supérieur, un alkyléthersulfate, un alkylphényléthersulfate polyoxyéthyléné, un sel sulfate d'alkylolamide et d'acide gras, un sel alkylétherphosphate, un sel alkylphosphate, un sel d'amine aliphatique, un sel d'ammonium quaternaire aliphatique, un sel de benzalkonium, le chlorure de benzéthonium, un sel de pyridinium, un sel d'imidazolinium, un alkyléther polyoxyéthyléné, un alkylphényléther polyoxyéthyléné, un copolymère séquencé de poly(oxyde d'éthylène)-poly(oxyde de propylène), un alkyléther polyoxyéthyléné polyoxypropyléné, un ester d'acide gras et de glycérol polyoxyéthyléné, l'huile de ricin polyoxyéthylénée, un ester d'acide gras et de sorbitan polyoxyéthyléné, un ester d'acide gras et de sorbitol polyoxyéthyléné, un ester d'acide gras et de polyéthylèneglycol, un monoglycéride d'acide gras, un ester d'acide gras et de polyglycérol, un ester d'acide gras et de sorbitan, un alcanolamide d'acide gras, un amide d'acide gras polyoxyéthyléné, une alkylamine polyoxyéthylénée, un oxyde d'alkylamine, la carboxybétaïne, un aminocarboxylate, et l'imidazolinium-bétaïne.

5. Utilisation selon la revendication 3 ou 4, dans laquelle l'excipient est un ou plusieurs excipients choisis dans le groupe constitué par:
les poudres minérales fines choisies dans le groupe constitué par l'argile kaolin, l'argile pyrophyllite, la bentonite, la montmorillonite, la terre de diatomées, l'oxyde de silicium hydraté synthétique, une argile acide, le talc, une argile, une céramique, le quartz, la séricite, la vermiculite, la perlite, la pierre d'Otani, l'anthracite, le calcaire, la houille, et la zéolite;
les composés inorganiques choisis dans le groupe constitué par le sel de table, les carbonates, les sulfates, les nitrates, et l'urée;
les matières organiques choisies dans le groupe constitué par les balles, le son, les carapaces de crabes, les carapaces de crevettes, la poudre de krill, la lie de riz, la farine de froment, les rafles de maïs, les coquilles de cacahuètes, la farine d'os, la farine de poisson, la poudre de lie, la farine de bois, le charbon de bois, le charbon de balles de riz, le charbon d'écorce, le charbon de coques de riz, le charbon végétal/charbon de bois, la mousse de tourbe, l'attapulgite, le fumier séché, le charbon activé, le tourteau, et l'amidon et ses hydrolysats;
les saccharides choisis dans le groupe constitué par le D-sorbitol, le lactose, le maltitose, la glucosamine, et les oligosaccharides;
les huiles choisies dans le groupe constitué par les huiles végétales, les huiles animales, et les huiles minérales; et
les polymères synthétiques solubles dans l'eau.

6. Utilisation selon l'une quelconque des revendications 3 à 5, dans laquelle ledit agent comprend la souche de *Talaromyces flavus* Y-9401 (FERM BP-10642) à raison de 1 à 90 % en masse, le tensioactif à raison de 1 à 15 % en masse, et l'excipient à raison de 0 à 98 % en masse.

7. Utilisation selon l'une quelconque des revendications 3 à 5, dans laquelle ledit agent comprend la souche de *Talaromyces flavus* Y-9401 (FERM BP-10642) à raison de 2 à 46 % en masse, le tensioactif à raison de 2 à 15 % en masse, et l'excipient à raison de 39 à 96 % en masse.

8. Utilisation selon l'une quelconque des revendications 1 à 7, dans laquelle ledit agent est formulé sous une forme quelconque parmi une poudre, un granule, une émulsion, une poudre mouillable, une formulation fluide, et un agent d'enrobage.

9. Méthode de lutte contre une maladie d'une plante, la méthode comprenant:
le traitement de semences, semis, organes végétaux, terreaux de semis, substrats de culture de semis, sols de culture, et/ou substrats de culture de la plante utilisant un agent comprenant la souche de *Talaromyces flavus* Y-9401 (FERM BP-10642);
dans laquelle la plante est choisie dans le groupe constitué par le chou; le soja; le maïs; les variétés de blé; et la pomme de terre; et
quand ladite plante est un chou, ladite maladie est provoquée par des champignons appartenant au genre *Rhizoctonia,*
quand ladite plante est le maïs, ladite maladie est provoquée par des champignons appartenant au genre *Pythium,*
quand ladite plante est une variété de blé, ladite maladie est provoquée par des champignons appartenant au genre *Pythium,*
quand ladite plante est le soja, ladite maladie est provoquée par des champignons appartenant au genre *Fusarium,* et
quand ladite plante est la pomme de terre, ladite maladie est provoquée par des bactéries appartenant au genre *Streptomyces* ou des champignons appartenant au genre *Rhizoctonia.*
